# EUROPÄISCHE PATENTSCHRIFT

(11) **EP 3 190 940 B1**
(45) Veröffentlichungstag und Bekanntmachung des Hinweises auf die Patenterteilung: **06.11.2019**
(21) Anmeldenummer: 15762603.7
(22) Anmeldetag: 10.09.2015
(51) Int. Cl.: A47L 13/10, A47L 25/08, A61L 2/10, C11D 17/04, D06F 35/00

(54) **VORRICHTUNG ZUR BEHANDLUNG VON FLECKEN SOWIE VERFAHREN ZUR BEHANDLUNG VON FLECKEN UNTER VERWENDUNG EINER SOLCHEN VORRICHTUNG**
DEVICE FOR TREATING STAINS AND METHOD FOR TREATING STAINS USING SUCH A DEVICE
DISPOSITIF DE TRAITEMENT DE TACHES AINSI QUE PROCÉDÉ DE TRAITEMENT DE TACHES À L'AIDE D'UN TEL DISPOSITIF

(30) Priorität: 10.09.2014 DE 102014218064
(43) Veröffentlichungstag der Anmeldung: 19.07.2017
(73) Patentinhaber: Henkel AG & Co. KGaA, 40589 Düsseldorf (DE)
(72) Erfinder: SPILL, Iwona, 12357 Berlin (DE); SCHMIEDEL, Peter, 40591 Düsseldorf (DE); DREJA, Michael, 41469 Neuss (DE); HEBERLEIN, Walter, 1060 Wien (AT)
(86) Internationale Anmeldenummer: PCT/EP2015/070696
(87) Internationale Veröffentlichungsnummer: WO 2016/038133

(56) Entgegenhaltungen:
- US-A- 2 862 221
- US-A1- 2005 120 756
- US-A1- 2007 011 835
- US-A1- 2007 189 834
- US-A1- 2014 059 790

## Beschreibung

Die vorliegende Erfindung betrifft eine Vorrichtung zur Behandlung von Flecken sowie ein Verfahren zur Behandlung von Flecken unter Verwendung einer solchen Vorrichtung.

Die Erfindung befasst sich mit der Entfernung von Flecken, insbesondere aus Textilien wie Kleidungsstücken, Bettwäsche, Tischwäsche und dergleichen. Oftmals entstehen lokal begrenzte Flecken beispielsweise durch Obst, Rotwein, Kosmetika etc. in Situationen, in denen es nicht möglich ist, das Textil unmittelbar zu wechseln und in die Wäsche zu geben. Dies kann beispielsweise auf Reisen oder im normalen Berufsalltag der Fall sein. Es ist wünschenswert, derartige Flecken lokal, zeitnah und unabhängig von einem regulären Waschgang behandeln zu können.

Darüber hinaus werden auch hartnäckige Flecken im normalen Waschgang in der Waschmaschine oft nur unzureichend beseitigt, insbesondere wenn niedrige Waschtemperaturen gewählt werden. Da viele Textilien aufgrund ihrer Materialzusammensetzung nicht bei höheren Waschtemperaturen waschbar sind, bedarf es in diesem Fall einer alternativen bzw. zusätzlichen Behandlung, um diese Flecken zu entfernen oder zumindest verblassen zu lassen.

In der US 7,596,974 B2 ist eine Vorrichtung und ein Verfahren zur sofortigen Fleckentfernung beschrieben, umfassend ein Flüssigkeitsreservoir zur Aufnahme einer Fleckenbehandlungsflüssigkeit sowie ein saugfähiges Material, wobei die Fleckenbehandlungsflüssigkeit auf den Fleck aufgebracht wird und nach Lösen oder teilweisem Lösen des Flecks mithilfe des saugfähigen Materials zumindest teilweise wieder abgenommen wird. Eine derartige Fleckbehandlung zeigt jedoch oft nur unzureichende Ergebnisse.

Insbesondere wirkt eine bestimmte Fleckenbehandlungsflüssigkeit aufgrund ihrer Zusammensetzung bevorzugt gegen einen bestimmten Typ von Flecken, während sie bei andersartigen Verunreinigungen kaum Wirkung zeigt.

Die zum Patent angemeldete DE 102013226835.1 beschreibt eine Waschmaschine mit einem Entfärbungsreservoir sowie ein Verfahren zum Waschen von Textilien mit einer solchen Waschmaschine. Das Entfärbungsreservoir weist ein Aktivierungsmittel, beispielsweise eine UV-Strahlungsquelle, auf, welches geeignet ist, innerhalb des Entfärbungsreservoirs einen Prozess zur Bildung freier Radikale in Gang zu setzen. Durch diese freien Radikale können Farbstoffe in der Waschflüssigkeit abgebaut werden. Eine Vorrichtung zur Behandlung von Flecken ist ebenfalls aus US-A-20050120756 bekannt.

Aufgabe der vorliegenden Erfindung ist die Bereitstellung von effizienten und einfach zu handhabenden Maßnahmen, mit welchen Flecken unterschiedlichster Herkunft in Textilien auch unabhängig von einem regulären Waschgang wirkungsvoll behandelt werden können.

Diese Aufgabe wird gelöst durch eine Vorrichtung gemäß Patentanspruch 1 sowie durch ein Verfahren gemäß Patentanspruch 12.

Vorteilhafte Ausgestaltungen und Weiterbildungen der Erfindung sind Gegenstand der Unteransprüche.

Gemäß Patentanspruch 1 handelt es sich bei der Erfindung um eine Vorrichtung zur Behandlung von Flecken.

Mit anderen Worten zeichnet sich die erfindungsgemäße Vorrichtung insbesondere dadurch aus, dass sie von einer Person mitführbar ist und somit in alltäglichen Situationen unabhängig von einem regulären Waschgang zum Einsatz kommen kann. Die Formulierung "eigentragfähig mitführbar" ist dabei im Sinne der Erfindung so zu verstehen, dass die Vorrichtung in ihren äußeren Abmessungen und in ihrem Gewicht derart bemessen ist, dass sie von einer einzelnen Person ohne großen Kraftaufwand mitgeführt werden kann, beispielsweise in einer Handtasche, in einem Rucksack oder auch in einer Hosentasche. Die Vorrichtung ist damit äußerst mobil und kann beispielsweise im Berufsalltag oder auf Reisen problemlos mitgeführt werden.

Es ist bekannt, dass Farbstoffe durch freie Radikale zersetzt werden können. Freie Radikale besitzen mindestens ein ungepaartes Elektron und sind aus diesem Grund äußerst reaktionsfreudig und dadurch kurzlebig (in der Regel < 1s). Sie sind in der Lage, mit Farbstoffen zu reagieren und diese so zu zersetzen. Beispielhaft sei die Zersetzung des Farbstoffes Acid Orange 7 genannt, welcher durch die Wechselwirkung mit freien Radikalen in farblose aromatische Nebenprodukte zersetzt wird, die ihrerseits durch Oxidation in aliphatische Säuren überführt werden.

Freie Radikale entstehen beispielsweise durch UV-Bestrahlung von chemischen Substanzen, welche Wasserstoffperoxid (H₂O₂) oder Titandioxid (TiO₂) enthalten. Durch die von der Strahlungsquelle emittierte UV-Strahlung wird das Wasserstoffperoxid bzw. Titandioxid aktiviert und als kurzlebige Produkte dieser Reaktion entstehen hoch reaktive Hydroxyl-Radikale (OH-Radikale), welche in der Lage sind, Farbstoffe zu zersetzen. Neben dem genannten Wasserstoffperoxid und Titandioxid sind zahlreiche weitere chemische Substanzen geeignet, welche sich durch UV-Bestrahlung unter Bildung freier Radikale aktivieren lassen. Die Konzentration der chemischen Substanzen in der Formulierung liegt dabei in einem Bereich zwischen 0,1 und 100%, vorzugsweise zwischen 5 und 30%. Die Formulierung kann Lösemittel umfassen.

Dieses Wechselwirkungsverhalten zwischen freien Radikalen und Farbstoffen macht sich die erfindungsgemäße Vorrichtung zur Behandlung von Flecken zu Nutze. Sie weist zu diesem Zweck das Reservoir zur Aufnahme einer chemischen Formulierung, beispielsweise Wasserstoffperoxid oder ein Wasserstoffperoxid enthaltendes Gemisch, auf, aus welchem die chemische Formulierung über die an dem Reservoir vorgesehene Auslassöffnung auf den zu behandelnden Fleck aufbringbar ist. Mit Hilfe der an der Vorrichtung angeordneten UV-Strahlungsquelle kann dann eine Bestrahlung der auf die Verunreinigung aufgebrachten chemischen Formulierung erfolgen, wodurch freie Radikale gebildet werden. Die gebildeten freien Radikale greifen die Farbstoffe in der zu behandelnden Verunreinigung an und zersetzen diese, so dass der Fleck verschwindet oder zumindest verblasst.

Als besonders vorteilhaft zeigt sich die hervorragende Wirkung der freien Radikale gegenüber verschiedensten Farbstoffen. Dadurch ist die erfindungsgemäße Vorrichtung äußerst vielseitig gegen Flecken unterschiedlichster Art einsetzbar, was sie gegenüber anderen bekannten Fleckenbehandlungsformulierungen auszeichnet.

Gemäß einer Ausgestaltung der Erfindung weist die Vorrichtung eine Länge von maximal 20 cm, eine Breite von maximal 10 cm und eine Höhe von maximal 6 cm auf. Mit durch diese Werte begrenzten Abmessungen ist die Vorrichtung ohne weiteres in herkömmlichen Taschen und Rucksäcken mitführbar. Innerhalb dieser Maximalabmessungen kann die Vorrichtung unterschiedlich geformt sein. So kann sie beispielsweise eine eher längliche oder eine eher quaderförmige Gestalt aufweisen. Sie kann jedoch auch rund oder ellipsoidal ausgebildet sein.

Nach der Erfindung ist die Vorrichtung stiftförmig ausgebildet, umfassend ein erstes und ein zweites Ende, wobei die Auslassöffnung des Reservoirs im Bereich des ersten Endes und die UV-Strahlungsquelle im Bereich des zweiten Endes der Vorrichtung angeordnet ist. Eine derartige stiftförmige Ausbildung erlaubt eine gute Handhabbarkeit der Vorrichtung sowohl im Gebrauch als auch beim Transport.

Hierzu beträgt die durch das erste und das zweite Ende festgelegte Länge einer solchen stiftförmigen Vorrichtung vorzugsweise 5 bis 20 cm, besonders bevorzugt 10 bis 15 cm. Der mittlere Durchmesser einer derartigen Vorrichtung liegt vorzugsweise zwischen 0,5 und 4 cm. Dabei kann die Vorrichtung über ihre gesamte Länge einen konstanten Durchmesser aufweisen und somit zylinderförmig ausgebildet sein, sie kann jedoch auch eine konische oder ellipsoidale äußere Form bzw. Mischformen hieraus aufweisen. Auch können abschnittsweise unterschiedliche Formtypen vorgesehen sein.

Die Erfindung sieht vor, dass die Vorrichtung mindestens zweiteilig ausgebildet ist, wobei das Reservoir in einem ersten Teil der Vorrichtung angeordnet ist und die UV-Strahlungsquelle in einem zweiten Teil der Vorrichtung angeordnet ist und wobei der erste und der zweite Teil der Vorrichtung über Verbindungsmittel miteinander verbindbar sind. Vorzugsweise sind der erste und der zweite Teil der Vorrichtung miteinander verschraubbar oder verrastbar.

Bei einer zwei- oder mehrteiligen Ausgestaltung der Vorrichtung kann das Reservoir einen Teil der Vorrichtung vollständig ausfüllen und identisch sein mit diesem Teil. Das Reservoir kann jedoch auch als abgeschlossenes Volumen innerhalb des entsprechenden Teils der Vorrichtung ausgebildet sein.

Bei der erfindungsgemäß vorgesehenen UV-Strahlungsquelle kann es sich um mindestens eine UV-Leuchtdiode, eine Anordnung von UV-Leuchtdioden vorzugsweise mit unterschiedlichen Wellenlänge, oder um eine UV-Quarzlampe handeln. Es sind jedoch auch andere UV-Strahlungsquellen wie Gasentladungslampen, Fluoreszenzlampen, Sonnenlicht oder Laser denkbar. In jedem Falle emittiert die UV-Strahlungsquelle ihre Strahlung erfindungsgemäß im Wesentlichen in eine Richtung von der Vorrichtung weg. Dies ist dahingehend zu verstehen, dass höchstens ein geringer Strahlungsanteil auf bzw. in die Vorrichtung zurückfällt.

Der bevorzugte Wellenlängenbereich der emittierten UV-Strahlung liegt zwischen 100 und 400 nm, besonders bevorzugt zwischen 250 und 400 nm. Für die Bildung von OH Radikale aus H2O2 ist zum Beispiel eine Wellenlänge von 254 nm geeignet. Radikal Bildung aus anderen Verbindungen kann ggf. durch andere Wellenlänge erzeugt werden. Die UV-Strahlungsquelle kann erfindungsgemäß eine Strahlung mit einem breiten Spektrum an Wellenlängen emittieren, es können jedoch beispielsweise auch mehrere Leuchtdioden verwendet werden, die jeweils Strahlung unterschiedlicher Wellenlänge emittieren.

Die Erfindung schlägt vor, dass das Reservoir ein Füllvolumen von 1 bis 50 ml aufweist. Grundsätzlich und bevorzugt kann das Reservoir dabei wiederbefüllbar gestaltet sein. Hierzu kann das Reservoir entweder im Bereich der Auslassöffnung oder davon beabstandet eine geeignete Öffnung aufweisen, durch welche die chemische Formulierung eingefüllt und nachgefüllt werden kann. Es fallen aber auch solche Vorrichtungen unter die Erfindung, welche nur einmalig bei der Herstellung mit einer entsprechenden chemischen Formulierung befüllt werden und bei vollständig entleertem Reservoir entsorgt werden. Schließlich kann es insbesondere bei der mindestens zweiteiligen Ausführung der Vorrichtung auch vorgesehen sein, dass lediglich der das Reservoir mit der chemischen Formulierung enthaltende Teil der Vorrichtung als austauschbares Wegwerfteil konzipiert ist, so dass der die UV-Strahlungsquelle enthaltende Teil mit immer wieder neuen Einmal-Reservoirteilen koppelbar ist.

Im Bereich des Reservoirs ist die Vorrichtung nach einer Ausgestaltung der Erfindung zusammendrückbar. Dies kann durch geeignete Materialwahl herbeigeführt werden und führt dazu, dass der Inhalt des Reservoirs durch entsprechenden Druck auf die Vorrichtung dosiert abgegeben werden kann.

Im Bereich der Auslassöffnung, insbesondere um diese herum, kann das Reservoir eine bürstenartige Struktur aufweisen, welche ein unmittelbares leichtes Einreiben der entnommenen Formulierung in das zu behandelnde Textil möglich macht und erleichtert.

Zur Abdeckung der UV-Strahlungsquelle und/oder der Auslassöffnung kann die Vorrichtung mindestens eine Schutzkappe aufweisen. Im Bereich der UV-Strahlungsquelle kann es sich um eine transparente, für UV-Strahlung durchlässige Abdeckung handeln, die auch im Betrieb nicht abgenommen wird und dem Schutz der Strahlungsquelle dient. Die Auslassöffnung kann von einer Schutzkappe abgedeckt sein, welche zur Entnahme der chemischen Formulierung abgenommen werden kann. Vorzugsweise weist die Schutzkappe zur Abdeckung der Auslassöffnung eine Dichtung auf, um ein unbeabsichtigtes Austreten der chemischen Formulierung aus der Vorrichtung zu vermeiden.

Die Schutzkappe kann beispielsweise mit einem Gewinde oder einer Verrastmöglichkeit versehen sein. Auch kann die Schutzkappe eine besondere Kindersicherung aufweisen, um zu verhindern, dass Kinder unbeabsichtigt in Kontakt mit der chemischen Formulierung kommen. Grundsätzlich kann die Schutzkappe im geöffneten Zustand vollständig abnehmbar sein oder über einen Verbindungssteg oder ähnliches an der Vorrichtung verbleiben.

An ihrem von der Vorrichtung beabstandeten Ende kann die mindestens eine Schutzkappe derart flach ausgeführt sein, dass die gesamte Vorrichtung bei geschlossener Schutzkappe auf dieser abstellbar ist. Hierdurch wird die Handhabbarkeit der Vorrichtung verbessert.

Zum Betreiben der UV-Strahlungsquelle umfasst die Vorrichtung erfindungsgemäß eine interne Batterieaufnahme und/oder einen Anschluss zum Herstellen einer Verbindung mit einer externen Spannungsquelle. Wird die Vorrichtung unterwegs eingesetzt, so ist sie durch Betrieb mit Batterien bzw. wiederaufladbaren Akkus flexibel und unabhängig von vorhandenen Spannungsquellen einsetzbar. Für den Einsatz zuhause, beispielsweise zur Vor- oder Nachbehandlung von hartnäckigen Flecken, kann die Vorrichtung auch über eine entsprechende Verbindung an eine externe Spannungsquelle angeschlossen werden. Hierzu kann die Vorrichtung einen entsprechenden Anschluss aufweisen.

Die UV-Strahlungsquelle wird vorzugsweise über einen Schalter ein- und ausgeschaltet, welcher außen an der Vorrichtung angeordnet ist.

Gemäß Patentanspruch 14 betrifft die Erfindung auch ein Verfahren zur Behandlung von Flecken unter Verwendung einer Vorrichtung nach einem der Ansprüche 1 bis 13, umfassend die Schritte:
- Aufbringen der chemischen Formulierung aus dem Reservoir auf einen zu behandelnden Fleck;
- Bestrahlung des solchermaßen mit der chemischen Formulierung versehenen Flecks mit UV-Strahlung;
- Gegebenenfalls Auswaschen des behandelten Flecks.

Der letzte Verfahrensschritt kommt insbesondere dann zur Anwendung, wenn das erfindungsgemäße Verfahren zur Vorbehandlung von hartnäckigen Flecken eingesetzt wird und sich nach erfolgter Vorbehandlung ein regulärer Waschgang anschließt. Selbstverständlich ist es jedoch auch möglich, mit dem erfindungsgemäßen Verfahren behandelte Flecken anschließend mit wenig klarem Wasser nachzuspülen, um möglicherweise verbliebene Reste der chemischen Formulierung zu entfernen.

Die UV-Bestrahlung wird erfindungsgemäß über einen Zeitraum von 1 bis 60 Minuten durchgeführt, vorzugsweise über einen Zeitraum von 5 bis 30 Minuten. Dabei ist der Grad der Fleckentfernung im Allgemeinen abhängig von der Dauer der Bestrahlung, so dass die Bestrahlungsdauer an die Intensität des zu behandelnden Flecks angepasst werden kann.

Auch die Menge der verwendeten chemischen Formulierung kann im Sinne der Erfindung an die Eigenschaften des zu entfernenden Flecks angepasst werden.

Im Folgenden wird die Erfindung anhand eines Ausführungsbeispiels und unter Bezugnahme auf die beigefügten Zeichnungen näher erläutert. Es zeigen:
- Figur 1:: ein Ausführungsbeispiel der erfindungsgemäßen Vorrichtung in perspektivischer Ansicht;
- Figur 2:: ein Längsschnitt durch die Vorrichtung aus Figur 1 in schematischer Darstellung.

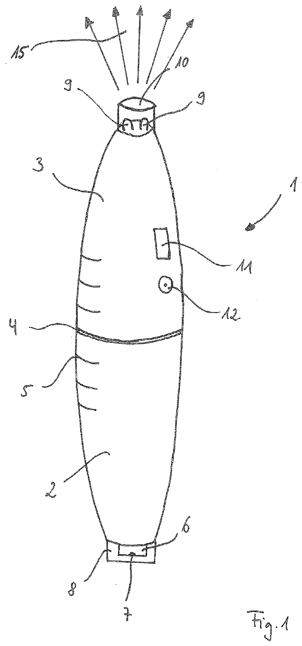

Figur 1 zeigt ein Ausführungsbeispiel einer im Ganzen mit 1 bezeichneten erfindungsgemäßen Vorrichtung zur Behandlung von Flecken. Die Vorrichtung ist zweiteilig ausgeführt und umfasst einen unteren ersten Teil 2 und einen oberen zweiten Teil 3. Der Teil 2 und der Teil 3 der Vorrichtung 1 sind miteinander verschraubt und weisen dazu an ihrem dem jeweiligen anderen Teil zugewandten Ende korrespondierende Gewinde auf. Im zusammengeschraubten Zustand stoßen die beiden Teile 2 und 3 im Bereich der Stoßkante 4 bündig aneinander.

Die Vorrichtung 1 hat eine Länge von 14 cm und weist annähernd die Gestalt eines Rotationsellipsoiden auf. Der Durchmesser der Vorrichtung 1 im Bereich der Stoßkante 4 beträgt etwa 3,5 cm.

Zur besseren Handhabbarkeit der Vorrichtung 1 weist diese eine mit 5 bezeichnete Riffelung auf, welche ein unbeabsichtigtes Wegrutschen der Vorrichtung 1 aus einer Hand eines Benutzers verhindern soll und der Vorrichtung eine gewisse Griffigkeit verleiht. Die Struktur 5 kann dabei, wie in Figur 1 dargestellt, lediglich abschnittsweise oder auch vollständig umlaufend ausgebildet sein.

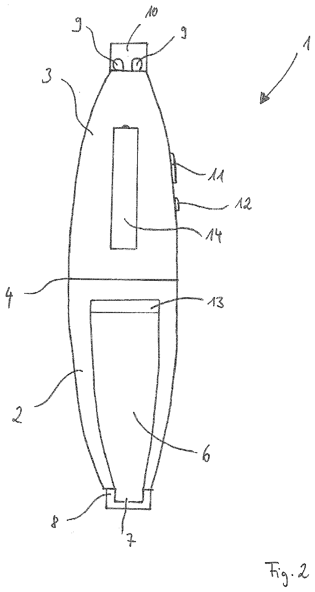

Innerhalb des unteren Teils 2 der Vorrichtung 1 ist ein Reservoir 6 angeordnet, von welchem in Figur 1 lediglich der unterste, aus der Vorrichtung 1 herausschauende Abschnitt sichtbar ist. Das vollständige Reservoir 6 ist aus der Schnittdarstellung in Figur 2 ersichtlich. Das Reservoir 6 dient der Aufnahme einer chemischen Formulierung, hier Wasserstoffperoxid, und hat ein Füllvolumen von 20 ml.

Das Reservoir 6 weist eine Auslassöffnung 7 auf, aus welcher die chemische Formulierung abgegeben werden kann. Zum Schutz der Auslassöffnung 7 ist darüber eine abnehmbare Schutzkappe 8 angeordnet, welche eine hier nicht dargestellte Dichtung aufweist. Die Schutzkappe 8 ist mit der Vorrichtung 1 verschraubbar und dichtet diese so gegen unbeabsichtigtes Austreten der chemischen Formulierung ab.

An ihrem dem Reservoir 6 abgewandten Ende ist die Schutzkappe 8 flach ausgeführt, so dass die gesamte Vorrichtung 1 bei aufgesetzter Schutzkappe 8 auf dieser abstellbar ist.

Am oberen Ende des oberen Teils 3 der Vorrichtung 1 sind zwei UV-Leuchtdioden 9 angeordnet, welche UV-Strahlung in einem Wellenlängenbereich von 240 bis 380 nm emittieren. Die UV-Leuchtdioden 9 emittieren die UV-Strahlung im Wesentlichen in eine Richtung von der Vorrichtung 1 weg, was in Figur 1 durch die Pfeilgruppe 15 angedeutet ist.

Die Leuchtdioden 9 werden über eine aus Figur 2 ersichtliche Batterie 14, welche innerhalb des oberen Teils 3 der Vorrichtung angeordnet ist, mit der nötigen Betriebsspannung versorgt. Der besseren Übersichtlichkeit halber wurde auf die Darstellung der Verbindungen zwischen UV-Leuchtdioden 9 und Batterie 14 verzichtet. Zusätzlich weist die Vorrichtung 1 eine Anschlussmöglichkeit 12 für den Anschluss an eine externe Spannungsquelle auf. Über einen auf der Außenseite der Vorrichtung 1 angeordneten Schalter 11 können die UV-Leuchtdioden 9 ein- und ausgeschaltet werden. Zur Abdeckung der UV-Leuchtdioden 9 weist die Vorrichtung 1 eine weitere Schutzkappe 10 auf, welche aus einem UV-durchlässigen Material besteht. Die Schutzkappe 10 dient damit dem Schutz der Leuchtdioden 9 und verbleibt auch während der Benutzung der Vorrichtung 1 in der beschriebenen Position. Genau wie die bereits beschriebene Schutzkappe 8 ist auch die Schutzkappe 10 an ihrem dem oberen Teil 3 abgewandten Ende derart flach ausgeführt, dass die gesamte Vorrichtung 1 darauf abstellbar ist.

Aus Figur 2 ist zu erkennen, dass das Reservoir 6 an seinem der Auslassöffnung 7 abgewandten Ende einen abschraubbaren Verschluss 13 aufweist. Zum Ein- bzw. Nachfüllen der chemischen Formulierung in das Reservoir 6 werden zunächst der erste Teil 2 und der zweite Teil 3 der Vorrichtung 1 auseinandergeschraubt. Sodann wird der Verschluss 13 des Reservoirs 6 abgeschraubt und die jeweilige Formulierung, hier Wasserstoffperoxid, aus einem Vorratsbehältnis in das Reservoir 6 eingefüllt. Der Verschluss 13 wird daraufhin wieder verschlossen und die beiden Teile 2 und 3 der Vorrichtung 1 miteinander verschraubt.

Zum Aufbringen der chemischen Formulierung auf einen zu behandelnden Fleck wird die Schutzkappe 8 abgenommen und eine gewünschte Menge der chemischen Formulierung durch leichtes Drücken auf die Vorrichtung 1 im Bereich des Reservoirs 6 aus diesem über die Auslassöffnung 7 entnommen und auf die Verunreinigung aufgebracht. Der untere Teil 2 der Vorrichtung 1 sowie das Reservoir 6 bestehen zu diesem Zweck aus einem leicht nachgiebigen Kunststoff.

Sobald die chemische Formulierung in der gewünschten Menge auf den zu behandelnden Fleck aufgebracht wurde, wird die Schutzkappe 8 wieder aufgesetzt und die Vorrichtung 1 um 180° gedreht. Über den Schalter 11 werden sodann die UV-Leuchtdioden 9 eingeschaltet und der mit der chemischen Formulierung getränkte Fleck mit UV-Strahlung bestrahlt. Die Dauer der Bestrahlung richtet sich dabei nach der Intensität und Hartnäckigkeit des zu entfernenden Fleckes. Durch die Bestrahlung mit UV-Strahlung wird das Wasserstoffperoxid aktiviert und als kurzlebige Produkte dieser Reaktion entstehen hoch reaktive Hydroxyl-Radikale (OH-Radikale), welche in der Lage sind, die in der Verunreinigung gebundenen Farbstoffe zu zersetzen. Nach einem Bestrahlungsintervall von typischerweise 5 bis 30 Minuten kann davon ausgegangen werden, dass der Fleck entfernt bzw. deutlich verblasst ist. Während dieses Bestrahlungsintervalls kann die Vorrichtung 1 auf der Schutzkappe 10 im Bereich des Flecks abgestellt werden, so dass insbesondere bei länger andauernder Bestrahlung das Halten der Vorrichtung 1 von Hand entfällt.

Nach erfolgreicher Behandlung des Flecks kann der behandelte Bereich des Textils beispielsweise mit klarem Wasser nachgespült werden, um mögliche Reste der chemischen Formulierung zu beseitigen. Sofern das oben beschriebene Verfahren zur Vorbehandlung eines Flecks eingesetzt wird, kann das Textil im Anschluss in die normale Wäsche gegeben werden.

Die Vorrichtung 1 sowie das mit dieser betriebene Verfahren stellen somit effiziente und einfach zu handhabende Maßnahmen dar, mit Hilfe derer unterschiedlichste Flecken in Textilien auch unabhängig von einem regulären Waschgang behandelt werden können. Insbesondere zeichnet sich die erfindungsgemäße Vorrichtung aufgrund ihrer Abmessungen und ihres geringen Gewichts dadurch aus, dass sie jederzeit überall hin mitgeführt werden kann.

## Patentansprüche

1. Vorrichtung zur Behandlung von Flecken, welche von einer Person eigentragfähig mitführbar ist, umfassend ein Reservoir (6) zur Aufnahme einer chemischen Formulierung, wobei das Reservoir (6) eine Auslassöffnung (7) zur Abgabe der chemischen Formulierung aufweist, und eine UV-Strahlungsquelle (9), welche ihre Strahlung im Wesentlichen in eine Richtung von der Vorrichtung weg emittiert, wobei die Vorrichtung stiftförmig ausgebildet ist, umfassend ein erstes und ein zweites Ende, wobei die UV-Strahlungsquelle (9) im Bereich des ersten Endes und die Auslassöffnung (7) des Reservoirs (6) im Bereich des zweiten Endes der Vorrichtung angeordnet ist, wobei die Vorrichtung mindestens zweiteilig ausgebildet ist, wobei das Reservoir (6) in einem ersten Teil (2) der Vorrichtung angeordnet ist und die UV-Strahlungsquelle (9) in einem zweiten Teil (3) der Vorrichtung angeordnet ist und wobei der erste und der zweite Teil (2, 3) der Vorrichtung über Verbindungsmittel miteinander verbindbar sind.

2. Vorrichtung nach Anspruch 1, **dadurch gekennzeichnet, dass** die Vorrichtung eine Länge von maximal 20 cm, eine Breite von maximal 10 cm und eine Höhe von maximal 6 cm aufweist.

3. Vorrichtung nach Anspruch 1, **dadurch gekennzeichnet, dass** der erste und der zweite Teil (2, 3) miteinander verschraubbar oder verrastbar sind.

4. Vorrichtung nach einem der Ansprüche 1 bis 3, **dadurch gekennzeichnet, dass** die UV-Strahlungsquelle (9) mindestens eine UV-Leuchtdiode oder eine Quarzlampe umfasst.

5. Vorrichtung nach einem der Ansprüche 1 bis 4, **dadurch gekennzeichnet, dass** die UV-Strahlungsquelle (9) UV-Strahlung in einem Wellenlängenbereich von 100 bis 400 nm, bevorzugt 250 bis 400 nm, emittiert.

6. Vorrichtung nach einem der Ansprüche 1 bis 5, **dadurch gekennzeichnet, dass** das Reservoir (6) ein Füllvolumen von 1 bis 50 ml aufweist.

7. Vorrichtung nach einem der Ansprüche 1 bis 6, **dadurch gekennzeichnet, dass** die Vorrichtung mindestens im Bereich des Reservoirs (6) zusammendrückbar ist.

8. Vorrichtung nach einem der Ansprüche 1 bis 7, **dadurch gekennzeichnet, dass** die Vorrichtung zur Abdeckung der UV-Strahlungsquelle (9) und/oder der Auslassöffnung (7) mindestens eine Schutzkappe (8, 10) aufweist.

9. Vorrichtung nach Anspruch 8, **dadurch gekennzeichnet, dass** die Schutzkappe (8) zur Abdeckung der Auslassöffnung (7) eine Dichtung aufweist.

10. Vorrichtung nach einem der Ansprüche 1 bis 9, **dadurch gekennzeichnet, dass** die Vorrichtung eine interne Batterieaufnahme umfasst und/oder einen Anschluss (12) zum Herstellen einer Verbindung mit einer externen Spannungsquelle aufweist.

11. Vorrichtung nach einem der Ansprüche 1 bis 10, **dadurch gekennzeichnet, dass** ein Schalter (11) vorgesehen ist, über welchen die UV-Strahlungsquelle (9) ein- und ausgeschaltet werden kann.

12. Verfahren zur Behandlung von Flecken unter Verwendung einer Vorrichtung nach einem der Ansprüche 1 bis 11, umfassend die Schritte:
- Aufbringen der chemischen Formulierung aus dem Reservoir (6) auf einen zu behandelnden Fleck;
- Bestrahlung des solchermaßen mit der chemischen Formulierung versehenen Flecks mit UV-Strahlung;
- Gegebenenfalls Auswaschen des behandelten Flecks.

13. Verfahren nach Anspruch 12, **dadurch gekennzeichnet, dass** die Bestrahlung mit UV-Strahlung über einen Zeitraum von 1 bis 60 Minuten, bevorzugt 5 bis 30 Minuten erfolgt.

## Claims

1. A device for treating stains which can be carried around on its own by a person, the device comprising a reservoir (6) for receiving a chemical formulation, wherein the reservoir (6) has an outlet opening (7) for dispensing the chemical formulation, and a UV radiation source (9) which emits its radiation substantially in a direction away from the device, wherein the device is pin-shaped, comprising a first and a second end, wherein the UV radiation source (9) is arranged in the region of the first end and the outlet opening (7) of the reservoir (6) is arranged in the region of the second end of the device, wherein the device is formed in at least two parts, wherein the reservoir (6) is arranged in a first part (2) of the device and the UV radiation source (9) is arranged in a second part (3) of the device, and wherein the first and the second part (2, 3) of the device can be interconnected via connecting means.

2. The device according to claim 1, **characterized in that** the device has a length of at most 20 cm, a width of at most 10 cm and a height of at most 6 cm.

3. The device according to claim 1, **characterized in that** the first and the second part (2, 3) can be screwed or locked together.

4. The device according to one of claims 1 to 3, **characterized in that** the UV radiation source (9) comprises at least one UV light-emitting diode or a quartz lamp.

5. The device according to one of claims 1 to 4, **characterized in that** the UV radiation source (9) emits UV radiation in a wavelength range of from 100 to 400 nm, preferably 250 to 400 nm.

6. The device according to one of claims 1 to 5, **characterized in that** the reservoir (6) has a filling volume of from 1 to 50 ml.

7. The device according to one of claims 1 to 6, **characterized in that** the device is compressible at least in the region of the reservoir (6).

8. The device according to one of claims 1 to 7, **characterized in that** the device comprises at least one protective cap (8, 10) for covering the UV radiation source (9) and/or the outlet opening (7).

9. The device according to claim 8, **characterized in that** the protective cap (8) for covering the outlet opening (7) has a seal.

10. The device according to one of claims 1 to 9, **characterized in that** the device comprises an internal battery holder and/or a terminal (12) for establishing a connection to an external voltage source.

11. The device according to one of claims 1 to 10, **characterized in that** a switch (11) is provided via which the UV radiation source (9) can be switched on and off.

12. A method for treating stains using a device according to one of claims 1 to 11, comprising the steps of:
- applying the chemical formulation from the reservoir (6) to a stain to be treated;
- irradiating the stain provided with the chemical formulation in this way with UV radiation; and
- optionally washing out the treated stain.

13. The method according to claim 12, **characterized in that** the irradiation with UV radiation takes place for a period of from 1 to 60 minutes, preferably 5 to 30 minutes.

## Revendications

1. Dispositif de traitement de taches pouvant être transporté par un individu, comprenant un réservoir (6) destiné à contenir une formulation chimique, le réservoir (6) ayant une ouverture de sortie (7) permettant de délivrer la formulation chimique, et une source de rayonnement UV (9) qui émet son rayonnement sensiblement dans une direction opposée au dispositif, le dispositif ayant une forme colonnaire, comprenant des première et seconde extrémités, la source de rayonnement UV (9) étant disposée dans la zone de la première extrémité, et l'ouverture de sortie (7) du réservoir (6) étant disposée dans la zone de la seconde extrémité du dispositif, le dispositif étant constitué d'au moins deux parties, le réservoir (6) étant disposé dans une première partie (2) du dispositif et la source de rayonnement UV (9) étant disposée dans une seconde partie (3) du dispositif, et les première et seconde parties (2, 3) du dispositif étant reliées entre elles par des moyens de liaison.

2. Dispositif selon la revendication 1, **caractérisé en ce que** le dispositif a une longueur maximale de 20 cm, une largeur maximale de 10 cm et une hauteur maximale de 6 cm.

3. Dispositif selon la revendication 1, **caractérisé en ce que** les première et seconde parties (2, 3) peuvent être vissées ou verrouillées ensemble.

4. Dispositif selon l'une quelconque des revendications 1 à 3, **caractérisé en ce que** la source de rayonnement UV (9) comprend au moins une diode électroluminescente UV ou une lampe à quartz.

5. Dispositif selon l'une quelconque des revendications 1 à 4, **caractérisé en ce que** la source de rayonnement UV (9) émet un rayonnement UV dans une plage de longueurs d'onde comprise entre 100 et 400 nm, de préférence entre 250 et 400 nm.

6. Dispositif selon l'une quelconque des revendications 1 à 5, **caractérisé en ce que** le réservoir (6) a un volume de remplissage compris entre 1 et 50 ml.

7. Dispositif selon l'une quelconque des revendications 1 à 6, **caractérisé en ce que** le dispositif est compressible au moins dans la zone du réservoir (6).

8. Dispositif selon l'une quelconque des revendications 1 à 7, **caractérisé en ce que** le dispositif de recouvrement de la source de rayonnement UV (9) et/ou de l'ouverture de sortie (7) présente au moins un capuchon de protection (8, 10).

9. Dispositif selon la revendication 8, **caractérisé en ce que** le capuchon de protection (8) présente un joint d'étanchéité pour recouvrir l'ouverture de sortie (7).

10. Dispositif selon l'une quelconque des revendications 1 à 9, **caractérisé en ce que** le dispositif comprend un logement pour batterie interne et/ou présente une borne (12) pour établir une connexion avec une source de tension externe.

11. Dispositif selon l'une quelconque des revendications 1 à 10, **caractérisé en ce qu'**interrupteur (11) est prévu pour allumer et éteindre la source de rayonnement UV (9).

12. Procédé de traitement de taches à l'aide d'un dispositif selon l'une quelconque des revendications 1 à 11, comprenant les étapes consistant à :
- appliquer la formulation chimique contenue dans le réservoir (6) sur une tache à traiter ;
- irradier, par rayonnement UV, la tache sur laquelle est appliquée la formulation chimique ;
- si nécessaire, laver la tache traitée.

13. Procédé selon la revendication 12, **caractérisé en ce que** l'irradiation par rayonnement UV s'effectue sur une période comprise entre 1 et 60 minutes, de préférence entre 5 et 30 minutes.
